# EUROPEAN PATENT SPECIFICATION

(11) **EP 2 515 846 B1**
(45) Date of publication and mention of the grant of the patent: **28.10.2015**
(21) Application number: 10771831.4
(22) Date of filing: 22.09.2010
(51) Int. Cl.: A61K 9/00, A61K 31/167, A61K 31/352, A61K 31/46, A61K 31/58, A61P 11/00

(54) **DRY POWDER COMBINATION OF TIOTROPIUM, BUDESONIDE AND A CROMOGLICIC ACID DERIVATIVE**
KOMBINATION VON TIOTROPIUM, BUDESONID UND CROMOGLYCIN SÄURE ALS PULVERINHALATION
COMBINAISON DE TIOTROPIUM, BUDESONIDE ET ACIDE CROMOGLICIQUE EN COMPOSITION DE POUDRE SÈCHE

(30) Priority: 25.12.2009 TR 200909789
(43) Date of publication of application: 31.10.2012
(73) Proprietor: Bilgic, Mahmut, 34220 Esenler-Istanbul (TR)
(72) Inventor: Bilgic, Mahmut, 34220 Esenler-Istanbul (TR)
(86) International application number: PCT/TR2010/000185
(87) International publication number: WO 2011/078815

(56) References cited:
- WO-A1-00/07567
- WO-A1-2009/029028
- WO-A2-02/36106
- VAN NOORD J A ET AL: "The efficacy of tiotropium administered via Respimat<(>R) Soft Mist(TM) Inhaler or HandiHaler<(>R) in COPD patients", RESPIRATORY MEDICINE, BAILLIERE TINDALL, LONDON, GB, vol. 103, no. 1, 1 January 2009 (2009-01-01), pages 22-29, XP025837396, ISSN: 0954-6111, DOI: DOI:10.1016/J.RMED.2008.10.002 [retrieved on 2008-11-20]

## Description

The present invention relates to a pharmaceutical composition containing tiotropium combined with a corticosteroid and an effective amount of a cromolyn derivative which is used for the treatment of respiratory disorders by inhalation route.

Tiotropium (Formula I) is an anticholinergic agent with chemical name (1α,2β,4β,7β)- 7-[(hydroxidi-2-thienylacetyl)oxy]-9,9-dimethyl-3-oxa-9-azoniatricyclo[3.3.1.0^{2,4}]nonane.

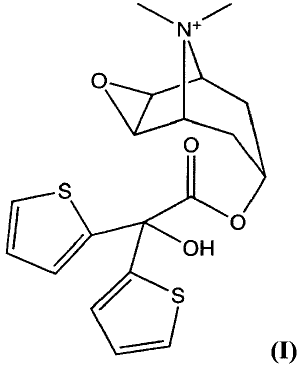

Tiotropium is described in European patent application numbered EP0418716 for the first time. In this patent document; processes for preparing tiotropium, pharmaceutical compositions containing tiotropium, long-acting, strong anticholinergic activity of tiotropium and use of it in the treatment of respiratory disorders, are defined.

Tiotropium is a long-acting, strong anticholinergic bronchodilator used by oral route for treating respiratory disorders. Tiotropium is in the form of dry powder contained in a capsule which is marketed under the trade name Spiriva and used by means of a dry powder inhalation device named Handyhaler. Tiotropium antagonises effect of acetylcholine via blocking cholinergic muscarinic receptor. Tiotropium is separated slowly from M1 and M3 receptors that cause broncho-construction, and it is separated rapidly from M2 receptors that inhibit release of acetylcholine from cholinergic nerve endings. This situation occurred in lung receptors demonstrates long acting bronchodilator activity of the drug.

Patent numbered WO 01/78743 is an example for using of tiotropium combined with corticosteroids. This patent document especially mentioned use of tiotropium combined with budesonide, and stated that a remarkable effect occurs in the treatment of respiratory disorder when tiotropium is used in combination with budesonide.

Patent numbered WO 0236106 is another example of the combination of anticholinergics (such as tiotropium or a salt thereof) with corticosteroids. The patent describes a medicament composition comprising anticholinergics (such as tiotropium or a salt thereof) and corticosteroids (such as budesonide), a method for production of said medicament and the use thereof for treating respiratory conditions. Inhalation route is a commonly preferred treatment method for respiratory disorders especially chronic disorders such as asthma and chronic obstructive pulmonary disorder (COPD) which threaten a large portion of the society. The reason is that drug reaches directly and rapidly to target area, accordingly lower doses of drug show desired effect by comparison with doses required for use via oral or parenteral route; and drug which is used in lower doses, show less side-effect than the drug administered via oral and parenteral route. Since the drugs used by inhalation route reach directly to the target area, they do not enter cardiovascular system and gastrointestinal problems which change drug effectiveness such as low solubility, low permeability, drug irritation, formation of unwanted metabolites and decreasing bioavailability due to food, are felt in minimum level.

Drug formulation used locally by inhalation route can be used by means of dry powder inhalers, metered dose inhalers or nebulization devices. All of these different drug delivey methods have pros and cons with respect to one another, dry powder inhalers come into prominence due to easy usage. However, there is still a need for developing different delivery methods for conveying the effective dosage to the lungs.

Lactose, which is a disaccharide, is generally used as a carrier in dry powder formulations developed for use via inhalation. The amount of lactose used is usually in the range of 10 mg to 25 mg, which is approximately 500-2500 times more than the amount of active agent, for a single dose. Although weight of the active agent is in micrograms, weight of lactose is in miligrams. However, a large amount of lactose used as a carrier in dry powder formulation caused side effects such as coughing, throat irritation, etc. Furthermore, use of dry powder formulations containing large amount of lactose by patients with allergy and/or lactose intolerance (sensivity to high-dose intake of lactose), causes symptoms such as nausea, stomach cramps, overfullness of the stomach, swelling of stomach, flatus, diarrhea, hives plaque. Because, 15 to 25% of each dose of the drugs administered by inhalation can reach the target area, the rest is mostly swallowed. Because of these drawbacks, there is a need for developing dry powder formulations containing tiotropium and a corticosteroid or pharmaceutically acceptable salts, hydrates, enantiomers, solvates, racemates, polymorphs, amorphous and/or crystal forms thereof, in terms of efficiency and safety.

Salts of cromoglicic acid and nedocromil are used for the treatment of bronchospasm in pharmacology. Because of this property, it is used for the treatment of asthma, allergic rhinitis, allergic and vernal conjunctivitis diseases.

Currently marketed products under the trade names "Intal" and "Cromohexal" provide 20 mg sodium cromoglicate per dose.

It is known that cromoglicic acid derivatives are used as a solubility enhancing agent in some aerosol formulations. For instance, US6475467 relates to use of a compound selected from cromoglicic acid and nedocromil derivatives in an ineffective amount in aerosol formulation that is in the form of suspension for increasing the dispersion of active agent in this suspension.

The present invention relates to use of salts of cromoglicic acid and/or nedocromil (B) in dry powder formulations containing tiotropium or a pharmaceutically acceptable derivative thereof (A) combined with budesonide (D). Initially, for the purpose of reducing the amount of lactose and alleviation of side effects caused by lactose, (B) has been added to the dry powder formulation which includes (A) and (D). But surprisingly, it has been found that when the amount of (B) is increased and (B) is used in an effective amount in the dry powder formulation containing (A) and (D), a synergistic effect of (B) is noticed in the formulation and an unexpected benefit occurs in the treatment of respiratory disease such as asthma, chronic obstructive pulmonary disease (COPD) and allergic rhinitis. Therefore, according to the present invention, when salts of cromoglicic acid and/or nedocromil (B) are used in dry powder formulations which include tiotropium or a pharmaceutically acceptable salt thereof (A) combined with budesonide or a pharmaceutically acceptable salt thereof (D), both required amount of carrier is reduced thus the side-effects caused by carrier are alleviated, and a synergistic effect is obtained for the treatment of respiratory diseases.

The present invention provides a medicament composition containing tiotropium or pharmaceutically acceptable derivatives such as salts, solvates, hydrates, polymorphs, amorphous and crystalline forms, free base thereof (A) combined with budesonide or pharmaceutically acceptable derivatives such as salts, hydrates, solvates, polymorphs, enantiomers, racemate, polymorphs, amorphous and crystalline forms thereof (D) as well as salt of cromoglicic acid and/or nedocromil in an effective amount, for use in the treatment of respiratory diseases.

The medicament composition in accordance with invention optionally contains a carrier in addition to the substances mentioned above.

Moreover, the invention provides use of (A), (D) and (B) defined hereinbefore in effective amounts for the preparation of a medicament which is used for the treatment of respiratory diseases especially asthma, chronic obstructive pulmonary diseases and allergic diseases.

While the medicament composition of the present invention was used, inventors encountered a problem of providing an effective amount of dose to the lungs which is a problem widely encountered during inhalation of dry powder formulations. In the prior art, various methods and devices are developed for dealing with this problem.

According to the present invention, it is found that the most suitable method for delivery of effective amount of dose to the target area is to prepare medicament composition in dry powder form and to inhale said medicament from a peelable blister strip. It is found that compared to the other methods for delivery of dry powder formulation comprising inhalation of dry powder formulation containing (A), (D), and (B) in effective amounts from capsule or from reservoir or from separate blister strips each of which contains a kind of active agent, the method for delivery of said dry powder formulation from a peelable blister strip minimize the drawbacks caused by adhesion force of micronized dry powder and the rate of medicament which reaches lungs increases.

In another aspect, the present invention provides a method comprising delivery of medicament composition containing (A), (D) and (B) in effective amounts and optionally a pharmaceutically acceptable carrier from the peelable blister strip by using dry powder inhaler for the treatment of respiratory diseases especially allergic disease.

In an embodiment of the invention, tiotropium (A) may be in the form of pharmaceutially acceptable salts and solvates, esters, free base, polymorphs thereof. Preferably, tiotropium bromide is used. All of crystal and amorphous forms of tiotropium, which shows different polymorphic forms, can be used within the scope of invention. Also, tiotropium used within the scope of invention can be in the form of anhydrate and hydrate. Preferably, tiotropium bromide anhydrate is used.

In an embodiment of the invention, corticosteroid, preferably budesonide (D), is in a form selected from a group comprising its pharmaceutically acceptable salts, hydrates, solvates, esters, polymorphs, enantiomers, racemates, free base, amorphous and crystalline forms thereof.

In an embodiment of the invention, salt of cromoglicic acid and/or nedocromil (B) can be selected from pharmaceutically acceptable salts thereof. Preferably, it can be either sodium cromoglicate and/or nedocromil sodium.

Dry powder formulation in accordance with the present invention administered via inhalation route optionally contains pharmaceutically acceptable carrier. The carrier can be selected from a group comprising arabinose, glucose, fructose, ribose, mannose, sucrose, trehalose, lactose, maltose, starches, dextran, sugar alcohols such as mannitol and saccharides.

The medicament composition in accordance with the present invention is in the form of micronized dry powder particles. The active agents present in said medicament composition have average particle size of 1 to 20 µm, preferably from 1 to 6 µm. The carrier present in said medicament composition typically has average particle size of not more than 300 µm, preferably not more than 210 µm. Salt of cromoglicic acid and/or nedocromil present in said medicament composition both as an active agent and as an excipient, that has average particle size in the range of 1 to 20 µm and the range of 10 to 300 µm.

The cavity volume of each blister in the peelable blister strip contained in the dry powder inhaler which is used for delivery of said medicament composition to the lung, is in the range of 20 to 30 mm³, preferably in the range of 21 to 25 mm³, most preferably in the range of 22 to 23 mm³.

A lid sheet and a base sheet of said blister strip are closed very tightly to provide impermeability by using suitable method.

According to the present invention, the lid sheet or the base sheet of the peelable blister strip consists of three layers. Two of these layers are polymeric layers and the other one is aluminium foil. Aluminium foil is used in both the lid sheet and the base sheet of the peelable blister strip to provide high humidity and gas protection because of that aluminium foil is conventionally used in both the lid sheet and the base sheet of blister strip for high humidty and gas protection. These layers must have the sufficient thickness which provides the protection for the stability of humidity sensitive dry powder formulation which is stored in blister cavity. Because of this reason, the thickness of aluminium foil that is used in the lid sheet and the base sheet of the peelable blister strip is in the range of 10 to 40 µm, preferably of 15 to 30 µm.

Two of the layers contained by the lid sheet and the base sheet of the peelable blister strip according to the present invention are polymeric layers. These polymeric layers may be made from either same or different polymers. The thickness of these polymeric layers depends on the type of polymeric substance used and its properties. Therefore, the thickness of each polymeric layer which is used in the lid sheet and the base sheet of said blister strip is in the range of 15 to 60 µm, preferably of 20 to 35 µm depending on the type of used polymer.

The inside layer of blister cavity of said blister strip which is in contact with dry powder formulation is polymeric layer because of the fact that aluminium foil causes adhesion of a part of dry powder formulation to inside layer of the cavity due to electrostatic forces, and hence cause uncontrolled dosing.

According to the present invention, the polymers used for forming polymeric layers are preferably selected from a group comprising thermo-plastic polymers such as polyethylene, polypropylene, polystyrene, polyolefin, polyamide, polyvinyl chloride, polyurethane.

The layers which are used for making up the lid sheet and the base sheet of said blister strip in accordance with present invention, are preferably same for each sheet, however the polymeric substances used for forming polymeric layers are preferably different from each other.

Moreover, each of the blister cavities which constitute the blister strip, can be different in shape as long as it has the properties defined above.

Devices used to inhale the dry powder formulation in accordance with the present invention may be multi dose inhalers present in the prior art. For this reason, the invention provides a medicament composition as it is mentioned before. In another aspect, the invention provides a method for delivery of medicament composition to patient's lungs effectively as it is mentioned before.

The medicament composition in accordance with the present invention containing active agents which is preferably in dry powder form is stored in the peelable blister strip and during inhalation 2 to 50 milligram of said medicament composition including one for each dose of (A), (D) and (B) is delivered to patients by using a multi dose inhaler, after each movement of the device.

In the medicament composition of present invention (A) is present in the amount of 1 to 50 µg, preferably 1 to 30 µg, (D) is present in the amount of 5 to 750 µg, preferably 5 to 550 µg, (B) is present in the amount of 1 to 50 mg, preferably 1 to 25 mg. Accordingly, the ratio of the total weight of (A) and (D) to the weight of (B) in said medicament composition is in the range of 1:5 to 1:1500. Additionally, in medicament composition pharmaceutically acceptable carrier can be used for the purpose of adjusting each dose of said medicament composition which is in dry powder form to the range of 5 to 50 mg.

The medicament composition in accordance with present invention can be used for the treatment of many respiratory diseases such as asthma, chronic obstructive pulmonary disorder (COPD) and allergic rhinitis. Accordingly, the respiratory diseases include but are not restricted to; allergic or non-allergic asthma in various phases, acute lung injury (ALI), acute respiratory distress syndrome (ARDS), exacerbation of airways hyperactivity, bronchiectasis, chronic obstructive pulmonary, airways or lung diseases (COPD, COAD or COLD) including emphysema and chronic bronchitis, pneumoconiosis, aluminosis, anthracosis, asbestosis, chalicosis, ptilosis, siderosis, silicosis, tabacosis and byssinosis. The treatment of said diseases may be prophylactic or symptomatic. In addition to this, the medicament composition in accordance with the present invention is used especially for the symptomatic treatment of asthma, allergic rhinitis and COPD.

This present invention, is explained with examples given below, but it is not restricted to these examples. Parts given in examples represent the weights of ingredients.

### Example 1

The medicament composition in dry powder form which is stored in a peelable blister strip to be delivered by using multi-dose inhaler and is prepared by mixing 18 parts of tiotropium bromide anhydrate, having average particle size of 1 to 5 µm, 200 parts of budesonide and 8000 parts of sodium cromoglicate, having average particle size of 1 to 5 µm, and 10000 parts of sodium cromoglicate having average particle size of not more than 300 µm, which are micronized in an air jet mill, and 5000 parts of a pharmaceuticall acceptable carrier such as lactose.

As given in the following example, tiotropium bromide anhydrate (A) can be extended tiotropium or pharmaceutically acceptable salts and all polymorphs thereof; budesonide (D) can be extended budesonide or pharmaceutically acceptable salts, enantiomers, polymorphs, solvates, hydrates, crystalline and amorphous forms thereof, sodium cromoglicate (K) extended salts of cromoglicic acid and pharmaceutically acceptable salts of nedocromil, more or less amount of pharmaceutically acceptable carrier may optionally be added, and, thus example 1 is repeated as following:

| | Amount of Tiotropium Bromide | Amount of Budesonide | Amount of A salt of cromoglicic acid salt |
|---|---|---|---|
| | (part) | (part) | (part) |
| Example 2 | 9 | 100 | 10000 |
| Example 3 | 9 | 100 | 12000 |
| Example 4 | 9 | 100 | 20000 |
| Example 5 | 9 | 100 | 30000 |
| Example 6 | 9 | 100 | 50000 |
| Example 7 | 12 | 200 | 10000 |
| Example 8 | 12 | 200 | 12000 |
| Example 9 | 12 | 200 | 20000 |
| Example 10 | 12 | 400 | 30000 |
| Example 11 | 12 | 400 | 10000 |
| Example 12 | 15 | 400 | 12000 |
| Example 13 | 15 | 400 | 20000 |
| Example 14 | 15 | 400 | 30000 |
| Example 15 | 15 | 400 | 10000 |
| Example 16 | 18 | 200 | 12000 |
| Example 17 | 18 | 200 | 20000 |
| Example 18 | 18 | 400 | 30000 |
| Example 19 | 18 | 400 | 10000 |
| Example 20 | 21 | 500 | 12000 |
| Example 21 | 21 | 500 | 20000 |
| Example 22 | 21 | 500 | 30000 |
| Example 23 | 21 | 500 | 10000 |
| Example 24 | 24 | 600 | 12000 |
| Example 25 | 24 | 600 | 20000 |
| Example 26 | 24 | 600 | 30000 |
| Example 27 | 24 | 750 | 10000 |
| Example 28 | 27 | 400 | 12000 |
| Example 29 | 27 | 400 | 20000 |
| Example 30 | 27 | 400 | 30000 |
| Example 31 | 27 | 400 | 10000 |
| Example 32 | 30 | 200 | 12000 |
| Example 33 | 30 | 200 | 20000 |
| Example 34 | 30 | 600 | 30000 |
| Example 35 | 30 | 600 | 10000 |

## Claims

1. A medicament composition containing combination of tiotropium or a pharmaceutically acceptable derivative thereof (A) and budesonide or a pharmaceutically acceptable salt thereof (D) for use in the treatment of respiratory disorder by inhalation route **characterized in that** said medicament composition contains an effective amount of pharmaceutically acceptable salt of cromoglicic acid and/or nedocromil (B).

2. A medicament composition according to claim 1, wherein tiotropium (A) is preferably tiotropium bromide.

3. A medicament composition according to claim 1, wherein tiotropium (A) is preferably tiotropium bromide anhydrate.

4. A mediacament composition according to claim 1, wherein budesonide (D) is in the form pure enantiomer, mixture of enantiomers or racemate.

5. A medicament composition according to any one of the preceding claims, wherein salt of cromoglicic acid (B) is sodium cromoglicate.

6. A medicament composition according to any one of claims 1 to 4, wherein salt of nedocromil (B) is nedocromil sodium.

7. A medicament composition according to claim 1, wherein said medicament composition used locally for the treatment of respiratory diseases by inhalation route contains micronized dry powder.

8. A medicament composition in dry powder form according to claim 7, wherein said medicament composition administered directly to airways by using a dry powder inhalation device.

9. A medicament composition according to claim 7, wherein active agents have average particle size of 1 to 20 µm, preferably 1 to 6 µm.

10. A medicament composition in dry powder form according to any one of the preceding claims, wherein tiotropium (A) is present in the amount of 1 to 50 µg, budesonide (D) is present in the amount of 5 to 750 µg and a salt of cromoglicic acid and/or nedocromil (B) is present in the amount of 1 to 50 mg for each dose and any amount of a pharmaceutically acceptable carrier for the purpose of adjusting each dose of medicament composition, which is in dry powder form, to the range of 5 to 50 mg.

11. A medicament composition in dry powder form according to claim 10, wherein the ratio of the total weight of tiotropium (A) and budesonide (D) to weight of a salt of cromoglicic acid (B) is in the range of 1:5 to 1:1500.

12. A medicament composition in dry powder form according to claim 7, wherein said medicament optionally contains a pharmaceutically acceptable carrier.

13. A medicament composition according to claim 12 wherein said medicament composition containing a pharmaceutically acceptable carrier selected from a group comprising monosaccharide, disaccharide, polysaccharide and oligosaccharide.

14. A medicament composition containing tiotropium or a pharmaceutically acceptable salt thereof (A), budesonide or a pharmaceutically acceptable salt, enantiomer or hydrate thereof (D), a salt of cromoglicic acid and/or nedocromil (B) and optionally pharmaceutically acceptable carrier by a dry powder inhaler for the treatment of respiratory diseases in which said medicament composition is stored in a peelable blister strip..

15. Use of tiotropium (A), budesonide (D) and a salt of cromoglicic acid and/or nedocromil (B) for the preparation of a medicament according to any one of preceding claims for the treatment of inflammatory or obstructive respiratory diseases.

## Patentansprüche

1. Eine Medikamentenzusammensetzung, die Kombinatin von Tiotropium oder ein pharmazeutisch annehmbares Derivat davon (A) und Budesonid oder ein pharmazeutisch annehmbares Salz davon (D) zur Verwendung bei der Behandlung von Störungen der Atemwege durch den Inhalationsweg enthält und **dadurch gekennzeichnet, dass** die genannte Medikamentenzusammensetzung eine wirksame Menge des pharmazeutisch annehmbaren Salzes von Cromoglycinsäure und / oder Nedocromil (B) enthält.

2. Eine Medikamentenzusammensetzung nach Anspruch 1, wobei Tiotropium (A) vorzugsweise Tiotropiumbromid ist.

3. Eine Medikamentenzusammensetzung nach Anspruch 1, wobei Tiotropium (A) vorzugsweise Tiotropiumbromid-anhydrat ist.

4. Eine Medikamentenzusammensetzung nach Anspruch 1, wobei Budesonid (D) in der Form des reinen Enantiomeren, der Mischung von Enantiomeren oder Racemate ist.

5. Eine Medikamentenzusammensetzung nach einem der vorhergehenden Ansprüche, wobei Salz der Cromoglicinsäure (B) Natriumcromoglicat ist.

6. Eine Medikamentenzusammensetzung nach einem der Ansprüche 1 bis 4, wobei Salz von Nedocromil (B) Nedocromilnatrium ist.

7. Eine Medikamentenzusammensetzung nach Anspruch 1, wobei die genannte Medikamentenzusammensetzung, die örtlich zur Behandlung von Atemwegserkrankungen durch Inhalationsweg verwendet wird, das mikronisierte Pulver enthält.

8. Eine Medikamentenzusammensetzung in trockener Pulverform nach Anspruch 7, wobei die genannte Medikamentenausammensetzung direkt auf die Atemwege durch die Verwendung eines Trockenpulver-Inhalationsgeräts verabreicht wird.

9. Eine Medikamentenzusammensetzung nach Anspruch 7, wobei die Wirkstoffe durchschnittliche Teilchengröße von 1 bis 20 µm, bevorzugt von 1 bis 6 µm haben.

10. Eine Medikamentenzusammensetzung in trockener Pulverform nach einem der vorhergehenden Ansprüche, wobei Tiotropium (A) in der Menge von 1 bis 50µg vorhanden ist; Budesonid (D) in der Menge von 5 bis 750 µg und ein Salz der Cromoglicinsäure und/oder Nedocromil (B) in der Menge von 1 bis 50 mg je Dosis und in jeder Menge eines pharmazeutisch annehmbaren Trägers vorhanden ist, um die einzelne Dosis der Medikamentenzusammensetzung, die in trockener Pulverform ist, auf das Bereich von 5 bis 50 mg einzustellen.

11. Eine Medikamentenzusammensetzung in trockener Pulverform nach Anspruch 10, wobei das Verhältnis des Gesamtgewichts von Tiotropium (A) und Budesonid (D) auf das Gewicht eines Salzes der Cromoglicinsäure (B) im Bereich von 1:5 bis 1:1500 liegt,

12. Eine Medikamentenzusammensetzung in trockener Pulverform nach Anspruch 7, wobei das genannte Medikament gegebenenfalls einen pharmazeutisch annehmbaren Träger enthält.

13. Eine Medikamentenzusammensetzung nach Anspruch 12, wobei die genannte Medikamentenzummensetzung, die einen pharmazeutisch annehmbaren Träger enthält, aus einer Gruppe bestehend aus Monosaccharid, Disaccharid, Polysaccharid und Oligosaccharid ausgewählt wird.

14. Eine Medikamentenzusammensetzung enthaltend Tiotropium oder ein pharmazeutisch annehmbares Salz davon (A), Budesonid oder ein pharmazeutisch annehmbares Salz, Enantiomer oder Hydrat davon (D), ein Salz der Cromoglicinsäure und/oder Nedocromil (B) und gegebenenfalls einen pharmazeutisch annehmbaren Träger durch einen trockenen Pulverinhalator zur Behandlung von Atemwegserkrankungen, bei denen die genannte Medikamentenzusammensetzung in einem abziehbaren Blisterstreifen gespeichert wird.

15. Verwendung von Tiotropiumbromid (A), Budesonid (D) und einem Salz der Cromoglicinsäure und/oder Nedocromil (B) zur Herstellung eines Medikaments nach einem der vorstehenden Ansprüche zur Verwendung bei der Behandlung von entzündlichen oder obstruktiven Atemwegserkrankungen.

## Revendications

1. Une composition de médicament contenant la combinaison de tiotropium ou un dérivé pharmaceutiquement acceptable de celui-ci (A) et le budésonide ou un sel pharmaceutiquement acceptable de celui-ci (D) pour utilisation dans le traitement des troubles respiratoires par la route d'inhalation **caractérisé en ce que** ladite composition de médicament contient une quantité efficace du sel pharmaceutiquement acceptable d'acide cromoglicique et/ou de nédocromil (B).

2. Une composition de médicament selon la revendication 1, dans laquelle, le tiotropium (A) est de préférence le bromure de tiotropium.

3. Une composition de médicament selon la revendication 1, dans laquelle, le tiotropium (A) est de préférence le bromure de tiotropium anhydre.

4. Une composition de médicament selon la revendication 1, dans laquelle, le budésonide (D) est sous la forme d'un énantiomère pur, un mélange d'énantiomères ou de racémates.

5. Une composition de médicament selon l'une quelconque des revendications précédentes, dans laquelle le sel d'acide cromoglicique (B) est le cromoglicate de sodium.

6. Une composition de médicament selon l'une quelconque des revendications 1 à 4, dans laquelle le sel de nédocromil (B) est le nédocromil de sodium.

7. Une composition de médicament selon la revendication 1, dans laquelle, ladite composition de médicament qui est utilisé localement pour le traitement, par inhalation, des troubles respiratoires contient une poudre micronisée sèche.

8. Une composition de médicament sous la forme d'une poudre sèche selon la revendication 7, dans laquelle, ladite composition de médicament est administrée directement dans les voies respiratoires en utilisant un dispositif d'inhalation de poudre sèche.

9. Une composition de médicament selon la revendication 7, dans laquelle, les agents actifs ont une taille moyenne de 1 à 20 µm, de préférence de 1 à 6 µm des particules.

10. Une composition de médicament sous la forme d'un poudre sèche selon l'une quelconque des revendications précédentes, dans laquelle le tiotropium (A) est présent dans la plage de 1 à 50 µm, le budésonide (D) est présent dans la plage de 5 à 750 µm et un sel d'acide cromoglicique et/ou nédocromile (B) est présent dans la plage de 1 à 50 mg pour chaque dose et un porteur pharmaceutiquement acceptable en quelque quantité suffisant afin d'ajuster chaque dose de la composition de médicament étant sous la forme de poudre sèche, à la plage de 5 à 50 mg.

11. Une composition de médicament sous la forme d'une poudre sèche selon la revendication 10, dans laquelle le rapport entre le poids total du tiotropium (A) et du budésonide (D) et le poids d'un sel d'acide cromologlicique (B) est dans la plage de 1:5 à 1:1500.

12. Une composition de médicament sous la forme d'une poudre sèche selon la revendication 7, dans laquelle ledit médicament contient éventuellement un porteur pharmaceutiquement acceptable.

13. Une composition de médicament selon la revendication 12, dans laquelle ladite composition de médicament contenant un porteur pharmaceutiquement acceptable est choisi dans un group comprenant des monosaccharides, des disaccharides, des polysaccharides et des oligosaccharides.

14. Une composition de médicament contenant du tiotropium ou un sel pharmaceutiquement acceptable de celui-ci (A), le budésonide ou un sel pharmaceutiquement acceptable, énantiomère ou un hydrate de celui-ci (D), un sel d'acide cromoglicique et/ou de nédocromil (B) et éventuellement un porteur pharmaceutiquement acceptable par un inhalateur de poudre sèche pour le traitement des troubles respiratoires, dans laquelle ladite composition de médicament est stocké dans une bande de blister pelable.

15. L'utilisation de tiotropium (A), budésonide (D) et un sel d'acide cromoglicique et/ou du nédocromil (B) pour la préparation d'un médicament selon l'une quelconque des revendications précédents afin d'utiliser dans le traitement des maladies inflammatoires ou respiratoires obstructives.
